# EUROPEAN PATENT APPLICATION

(11) **EP 4 279 575 A1**
(43) Date of publication of application: **22.11.2023**
(21) Application number: 22174630.8
(22) Date of filing: 20.05.2022
(51) Int. Cl.: C12M 3/06, B01L 3/00, C12M 1/26, C12M 1/00

(54) **DEVICE AND METHOD FOR PARTICLE ISOLATION**

(71) Applicant: Albert-Ludwigs-Universität Freiburg, 79098 Freiburg (DE); Hahn-Schickard-Gesellschaft für angewandte Forschung e.V., 78052 Villingen-Schwenningen (DE)
(72) Inventor: Zieger, Viktoria, 68794 Oberhausen-Rheinhausen (DE); Kartmann, Sabrina, 79109 Freiburg (DE); Frejek, Daniel, 79232 March (DE)
(74) Representative: Stöckeler, Ferdinand

(57) **Abstract**

A device for particle isolation comprises a reservoir to contain a liquid including particles and a channel with an inlet and an outlet. The inlet is configured to be fluidically coupled to the reservoir containing the liquid including particles. The device comprises further a pressure generator configured to generate a pressure gradient in the channel by which liquid including a single particle is aspirated from the reservoir through the inlet of the channel against the force of gravity and to cause liquid to be output through the outlet of the channel. Additionally, the device comprises an actuator configured to cause a relative movement between the particles and the inlet of the channel so as to bring a single particle in a vicinity of the inlet to be aspirated through the inlet.

## Description

### Technical Field

Embodiments according to the invention related to a method and a device for particle isolation, like a micro-particle isolation platform.

### Background of the Invention

While the use of high-throughput screening platforms (hereinafter referred to as HTS platforms) is already extensively established and regularly used for the screening of active molecules in large-scale pharmaceutical research, highly parallel methods have not yet gained any relevant significance in the near-patient environment. This is largely due to the high system costs of HTS platforms and the lack of integration of the generated results into clinical applications: Early drug screening is limited to active ingredient libraries that are tested in various concentrations on established tumor cell lines. Successful candidates are then examined in greater depth and, if necessary, fed into the pharmaceutical development process. Typically, these steps, biological validation, chemical-pharmaceutical modification, and the translational study phase take seven to ten years (Skardal, 2016). In this process, the success rate of active ingredient development is also very low, as the cell lines used are very inadequate models of a physiological tumor. Consequently, HTS increasingly attempts to conduct experiments on 3D cultures, spheroids and organ models (Eglen, 2015; Esch, 2015). However, the necessary high-throughput technologies to process a significant number of spheroids or organ models in a fully automated manner have been lacking so far. Only in recent years, innovative technologies and companies have appeared on the market that promise first solutions (Zhang, 2017). The herein discussed invention therefore represents an important contribution with regard to the establishment of such high-throughput platforms for 3D cell culture.

In the context of the herein discussed invention, a solution for depositing a controllable number of 3D cell culture models, and more precisely spheroids or organoids, in a reaction chamber (well of a microtiter plate) with high throughput is presented.

To date, only three automated single spheroid approaches have been published, including the technology developed at IMTEK (Gutzweiler, 2017). The published approaches for single-spheroid deposition are based on a print head, which controls predetermined coordinates with an axis robot. The document of 'Cyfuse Biomedical K. K., 2019' discloses a pick-and-place process with a cannula that aspirates spheroids and can deposit them again. The document of 'Lang, 2010' discloses a microfluidic dual-chamber separator that uses a pestle to move individual spheroids from the loading chamber to the injection chamber, from where the same can be deposited into droplets. The document of 'Gutzweiler, 2017' shows a non-contact dosage of single-spheroid droplets, which are already detected in the nozzle by means of optical detection. Vacuum aspiration separates droplets that do not meet the detection criteria.

The *Regenova Bio 3D Printer* (Cyfuse Biomedical K.K., Tokyo, Japan) uses a pick-and-place method (Cyfuse Biomedical K.K., 2019). Here, a cannula uses a vacuum to fix individual spheroids from a culture vessel at its inlet. These are then moved by a three-axis robot to the deposition site, where the corresponding spheroid can be deposited. This method is also used by Ayan et al. (2021), who also use a glass cannula to generate a vacuum in order to aspirate and selectively place spheroids (Ayan, 2020). It is important to generate only a minimal aspiration force, as this will not break the spheroids. However, the maximum force could vary greatly depending on the type of cell affected and would accordingly have to be adapted for different patient biopsies and cancer types. If the uptake site, where the spheroids are aspirated, and the delivery site, where the spheroids are delivered, are too far apart, fast transport velocities are needed to enable high throughput. However, this is not recommended with regard to evaporation and desiccation of the biological samples.

The *Spheroid seeding device* (Department of Orthopaedic Surgery, University of Otago Christchurch, New Zealand) is able to separate individual spheroids and deposit them again in a controlled manner using a three-axis robot (Lang, 2010). For this purpose, two separate fluidic chambers are used, which are connected via a channel. Between the chambers, spheroid isolation takes place in a fluidic channel. This transfers individual spheroids from the loading chamber to the injection chamber, from where the same can be deposited. This approach is difficult to implement when spheroid sizes vary, which could lead to occlusions in the chambers, as well as insufficient isolation. In addition, the design of the fluidic chambers with the different pestles for moving spheroids represents a challenge for the implementation as a cost-effective disposable product or as a sterilizable product.

The scalable single-spheroid deposition system (Institut für Mikrosystemtechnik IMTEK, University of Freiburg, Germany) uses the commercially available, non-contact microdosage device *PipeJet*^{®} (Biofluidix GmbH, Germany) in combination with a specially developed optical single-spheroid detection (Gutzweiler, 2017). Spheroids are detected within the nozzle and only tube droplets containing exactly one spheroid are deposited in a locally controlled manner. The system can select according to geometric criteria such as size and shape of the spheroids. Droplets that do not meet the set criteria are aspirated by a vacuum system. All components that come into contact with liquid are sterilizable disposable products. However, this approach has shown that very high loss rates are generated by vacuum aspiration and sedimentation problems in tubes leading to the nozzle cause severe occlusion problems.

According to the prior art, there are some efforts to technically realize the dosing of small quantities of liquids as well as the isolated dispensing of particles.

Patent TWI7184258 describes a fluid dispenser in which fluid is taken up by capillary forces, which can then be discharged by a fluid dispenser (Taiwan Patent No. TWI718425B, 2017). An essential feature is that the suction device is directed in the same direction as the fluid dispenser and projects beyond the same. The capillary suction device is a tube with a tapered, hydrophilic interior. According to the embodiment of the present patent, the spectrum of targeted dispensing of different fluid volumes, e.g. in the field of microbiology, is to be expanded.

Patent EP2577254A1 describes a device for dispensing particles in a free-flying droplet (Europe Patent No. EP2577254A 1, 2010). The particle suspension is placed in an unbranched one-way channel before the droplets are generated by a piezo-driven device. Empty droplets are detected by an observation device at the one-way channel and directed to a reject position by a special device. This makes it possible, for example, to deposit cells in an isolated manner in microtiter plates. However, a problem with this approach is that no solution is given as to how the particle suspension enters the one-way channel. In order to avoid channel occlusions and thus minimize the loss rate, a certain degree of isolation is required, especially for larger and stiffer particles, such as spheroids compared to single cells.

Patent AU2008232516 82 presents a device for taking a tissue sample (Australia Patent No. AU2008232516 B2, 2014). It is a device which, by means of an aspiration force, transports fluid containing various tissue particles through a housing. The device houses two filters, the first filter with a large pore size sorting out coarse components from the fluid and the second filter filters particles of a certain size. Although a solution for automated filling of a liquid handling platform via an aspiration force is given, this approach does not offer a solution for isolating the particles in the fluid. Due to the different filters, large portions of the tissue sample are lost, which then have to be reprocessed at great expense.

Cytostatics are an essential pillar of modern cancer therapy and are indispensable for palliative treatment, especially in advanced stages. Despite scientifically sound and highly complex sequence and parallel regimens of different active ingredients, however, treatment providers as well as patients face major challenges when patients react differently to active ingredients or even develop resistances to the cytostatics used. Even if the collective human genomes are more similar (>99%) than different (<1%), these small differences can have far-reaching consequences. Genome and epigenome-wide, transcriptomic and proteogenomic profiling techniques are now available that allow detailed characterization and categorization of cancer at an individual level, providing new opportunities for really tailored approaches for treatment and patient care (Bode, 2017).

In recent years, three-dimensional (3D) cell cultures in the form of spheroids or organoids produced from cell lines or human tissue have gained in significance. In cancer research, Solid tumors are no longer understood as collections of homogeneous cell mutants, but as the result of a microevolutionary process (Alberts, 2014). One obstacle to the development of novel treatment methods is the challenge of transferring scientific findings from the laboratory environment to the patient, which is mainly due to the fact that many cancer models poorly represent the patient's tumor and many drugs that perform well in cancer models ultimately fail in clinical studies (Ghosh, 2011; Stintzing, 2016). Animal cancer models have undoubtedly provided important insights into the basis of cancer, but they are very time consuming and not necessarily predictive for the human organism, as they do not faithfully reproduce pathogenic processes in patients. For example, the histological complexity and genetic heterogeneity of human cancers is typically not reflected in genetically modified mouse models of cancer (Stintzing, 2016; Caponigro, 2011).

A major problem with the spheroid handling platforms known to date is the isolation of a spheroid from a suspension without limiting flexibility and process control while keeping loss rates as low as possible.

Therefore, it is desired to provide a flexible concept with high process control, which makes a better compromise between improving an isolation of single particles from a suspension, improving a transport of a single particle to a destined position and reducing loss rates. The aim of the invention is to provide a liquid handling platform, which automatically, sterile, flexible, with high process control and high throughout reliably separates micro particles with a low loss rate.

This is achieved by the subject matter of the independent claims of the present application.

Further embodiments according to the invention are defined by the subject matter of the dependent claims of the present application.

The herein discussed approach to personalized medicine aims to minimize the use of animal testing by working not on (murine) 2D cell culture, but in a much more targeted way with 3D cell culture models (spheroids, organoids) from samples of patients. Patient-specific spheroids, e.g., tumor spheroids, can be used to gain important insights for individual treatment and for the development of new drugs in general. This means that animal testing can be used in a more targeted manner in (pre)clinical research and drug development in the future or, if possible, can even be avoided. With the aid of an automated processing platform based on a liquid handling system, spheroids are to be placed individually and in a controlled manner in individual wells of microtiter plates and subsequently treated with different chemotherapeutic active ingredients and concentrations. Automated spheroid handling is to compensate the current drawbacks of the prior art. The spheroid handling methods used to date for translational clinical research involve a large amount of manual labor. This limits the number of possible *in vitro* experiments and the number of technical replicates and results in limited statistical significance.

In summary, it can thus be stated that the herein discussed invention goes substantially beyond the prior art presented with respect to the following aspects:
- Miniaturization, parallelization, and automation with *in vitro* experiments with spheroids and organoids with high-throughput.
- The platform is also suitable for HTS in terms of throughput and automation like no other microfluidic technology.
- High degree of flexibility and process control is enabled by guiding specifically selected shperoids/organoids from a reservoir to a target position.

### Summary of the Invention

In accordance with an aspect of the present invention, the inventors of the present application realized that one problem encountered when trying to guide single particles to a target position stems from the fact that the particles sediment. According to this aspect of the present application, this difficulty is overcome by aspirating a single particle against the force of gravity. This is based on the idea that such a transport counteracts the sedimentation of particles and prevents therefore an occlusion of a channel through which the particles are transported. Especially, the active aspiration is advantageous, since the applied force overcomes passive forces acting onto a particle and it enables a faster transport of particles compared to a passive transport, like a transport using a capillary force. The usage of an active aspiration acting against the force of gravity results in an efficient transport of particles through a channel and enables a high-throughput. Further, the inventors found that it is highly efficient to use the same mechanism or device for aspirating particles through an inlet of a channel and for outputting liquid with or without a single particle through an outlet of the channel. This is based on the idea that, for example, a suction directed in an output direction can be applied at the output of the channel resulting in an outputting of fluid through the outlet and an aspirating of fluid through the inlet. Such a suction applies enough force onto a single particle to be aspirated through the inlet against a force of gravity and at the same time the applied force is small enough to not destroy or break the particle. Additionally, this enables a small system, since it is not necessary to provide separate devices or mechanisms for the aspiration and the outputting. A further advantage is the usage of a channel for a transport of a particle from a reservoir to a target position, this is because of the particle being suspended in a liquid during the whole transport. Thus, the channel prevents evaporation and desiccation during the transport and allows therefore also long distances between the reservoir and a target position and a fast transportation of the aspirated particle. Even further, the device uses a relative movement between the particles in the reservoir and the inlet of the channel to enable the aspiration of single particles through the inlet and, for example, to allow a specific selection of the particle to be aspirated. Thus, it is possible to transport single particles individually to their respective target position. The invention is a concept for separating particles, like cells, spheroids or organoids in a liquid medium in a reservoir by individually aspirating the particles from the reservoir, passing them through a thin tube or a capillary and transporting them to a target position.

Accordingly, in accordance with this aspect of the present application, a device for particle isolation comprises a reservoir to contain a liquid including particles and a channel with an inlet and an outlet. The inlet is configured to be fluidically coupled to the reservoir. For example, the channel might immerse into the reservoir with a channel portion comprising the inlet. The channel portion comprising the inlet may immerse into the liquid including the particles in the reservoir. Alternatively, the channel might be fixed to the reservoir. Further, the device comprises a pressure generator configured to generate a pressure gradient in the channel by which liquid including a single particle is aspirated from the reservoir through the inlet of the channel against the force of gravity. Additionally, the pressure generator is configured to cause liquid to be output through the outlet of the channel. The pressure generator might be a pump, a piezo-driven plunger or a thermal droplet generating device. Additionally, the device comprises an actuator configured to cause a relative movement between the particles and the inlet of the channel to bring a single particle in a vicinity of the inlet to be aspirated through the inlet.

According to an embodiment, a channel portion comprising the inlet might be arranged in an angle of at least 10° with respect to a bottom surface of the reservoir. This enables, for example, an aspiration of a particle through the inlet of the channel against the force of gravity. The inventors found that it is especially advantageous, if the channel is arranged in an angle of 30° or 90° with respect to the bottom surface.

According to an embodiment, the actuator is configured to move the reservoir and/or the inlet of the channel so as to bring the single particle, preferably a sedimented particle, in a vicinity of the inlet to be aspirated through the inlet. The single particle, for example, is a particle sedimented on a bottom surface of the reservoir. The actuator, for example, is a motorized positioner, e.g., a three-axis stage or robot, configured to move the reservoir with the single particle to bring the single particle in the vicinity of the inlet to be aspirated through the inlet. Alternatively, the actuator, for example, is a motorized positioner, e.g., a three-axis stage or robot, configured to move the inlet of the channel to bring the single particle in the vicinity of the inlet to be aspirated through the inlet. According to a preferred embodiment, the actuator comprises a first actuator configured to move the reservoir in two dimensions, e.g., a first dimension representing a x-direction and a second dimension representing a y-direction, and a second actuator configured to move the channel in a third dimension, e.g., in z-direction, perpendicular to a plane, e.g. a xy-plane, defined by the two dimensions, i.e. defined by the first dimension and the second dimension. For example, the first actuator and the second actuator are configured to bring the single particle specifically/purposefully in the vicinity of the inlet to be aspirated through the inlet. The first actuator, for example, moves the reservoir to bring a specific particle to the same (x, y)-position as the inlet of the channel and the second actuator moves the channel to reduce a height difference between the specific particle and the inlet of the channel. This combined movement of the reservoir and of the channel brings the specific particle efficiently in near proximity to the inlet to aspirate same through the inlet. The movement of the reservoir and/or of the inlet of the channel enables to selectively aspirate particles, especially sedimented particles, with the device.

According to an embodiment, the actuator is configured to induce mixing liquid flows in the reservoir to prevent particles from sedimenting in the reservoir and to bring the particles individually in the vicinity of the inlet of the channel. For example, the actuator may be a pump configured to induce the mixing liquid flows or a vibration device configured to vibrate the reservoir to induce the mixing liquid flows or a magnetic device configured to move magnetic particles in the reservoir to induce the mixing liquid flows. The caused mixing liquid flows move the particles within the liquid in the reservoir. The particles are lifted to the inlet of the channel to be aspirated. With this type of actuator the particles can be brought easily and efficiently to the inlet of the channel. Additionally, the mixing liquid flows may prevent the particles from aggregating and enable the aspiration of single particles through the inlet of the channel.

According to an embodiment, the reservoir is a reservoir channel within a microchip and the inlet is fluidically coupled to an end portion of the reservoir channel. The reservoir channel might represent a microchannel, which represents in microtechnology a channel with a diameter below 1 mm. The actuator, e.g., a flow generator, may be configured to cause a flow of the liquid in the reservoir channel to bring the particles individually in the vicinity of the inlet of the channel. For example, the actuator is configured to cause the flow of the particles to transport the particles through the reservoir channel lined up one after the other to the inlet of the channel. The actuator might be configured to apply sheath fluids in a laminar flow to focus the particles in a center of the flow or stream. Optionally, the reservoir channel might have a special shape, like a meander shape, to enable an individualization of the particles in the reservoir channel by the flow caused by the actuator.

According to an embodiment, the device comprises a reservoir monitoring device, e.g., a camera and/or a microscope and/or an impedance sensor, configured to monitor the reservoir, e.g., a part of the reservoir or the whole reservoir, to obtain information, i.e., first information, about a position of the single particle in the reservoir, e.g., a reservoir position. It is also possible that the reservoir monitoring device is configured to obtain further information, like positions of other particles, number of particles, size of particles, shape of particles etc. The actuator is configured to cause the relative movement to bring the single particle in the vicinity of the inlet based on the information about the position of the single particle. For example, the actuator is configured to move the inlet of the channel to the position of the single particle. In this case the actuator might be a three-axis motorized positioner, like a stage or a robot, configured to move a part of the channel comprising the inlet relative to the single particle in the reservoir. Alternatively, the actuator is configured to move the reservoir relative to the part of the channel comprising the inlet in order to position the reservoir, so that the inlet of the channel is at the position of the single particle. In this case the actuator might be a three-axis motorized positioner, like a robot or a stage, configured to move the reservoir with the single particle at the position defined by the information about the position of the single particle. Alternatively, it is also possible that the actuator comprises a first actuator configured to move the reservoir and a second actuator configured to move the channel, wherein the actuator uses the information about the position of the single particle to control the first actuator and the second actuator to bring the single particle in the vicinity of the inlet. This feature enables a selective aspiration of individual particles.

According to an embodiment, the reservoir monitoring device is further configured to obtain information, e.g., second information, about a size and/or shape of particles positioned in a monitoring area of the reservoir monitoring device. The device may further comprise a processor configured to determine for each of the particles positioned in the monitoring area a respective particle type based on the information about the size and/or the shape of the particles positioned in the monitoring area. The processor may be configured to select from the particles positioned in the monitoring area a particle of a predetermined particle type as the single particle to be aspirated and provide the information about the position of the single particle to the actuator. In case of different types of particles being suspended in a liquid in the reservoir, it is therefore possible to sort the particles by aspirating only particles of a predetermined type.

According to an embodiment, the reservoir monitoring device is configured to obtain information about positions of particles positioned in a monitoring area of the reservoir monitoring device. The device may further comprise a processor, e.g., the processor described above, configured to determine distances between the particles positioned in the monitoring area based on the information about the positions of the particles positioned in the monitoring area. The processor may be configured to select from the particles positioned in the monitoring area the single particle to be aspirated based on the determined distances between the particles and provide the information about the position of the single particle to the actuator. For each neighboring particle of the single particle to be aspirated, a respective distance between the single particle to be aspirated and the respective neighboring particle, for example, has to be equal to or greater than a predetermined threshold, e.g., a predetermined distance threshold. For example, the processor is configured to select the single particle to be aspirated, by checking whether the distances between a first particle and its neighboring particles are all equal to or greater than the predetermined threshold and by selecting the first particle as the single particle to be aspirated, if all distances between the first particle and its neighboring particles are greater than the predetermined threshold. The predetermined threshold, for example, is preset. The predetermined threshold may depend on a type of the particles to be aspirated, the dimension of the cross-section of the channel and/or the pressure gradient caused by the pressure generator. The predetermined threshold may be equal to 50 µm, 100 µm, 250 µm, 300 µm, 500 µm or 1 mm. By checking the distances between the particles it is ensured that only one single particle is aspirated through the inlet and not additionally neighboring particles.

According to an embodiment, the device comprises a monitoring device, e.g., a camera and/or a microscope and/or an impedance sensor, configured to monitor the vicinity of the inlet of the channel, e.g. the inlet, or a part of the channel comprising the inlet, and the surroundings of the inlet within the reservoir. The monitoring device might correspond to the reservoir monitoring device described above. The monitoring device might be configured to obtain information on whether a single particle is present in the vicinity of the inlet and/or information about size and/or shape of the single particle in the vicinity of the inlet and/or information whether the single particle is aspirated through the inlet and/or to obtain information about the time instant at which the single particle is aspirated through the inlet. The device further comprises a controller configured to control the pressure generator to cause the pressure gradient in the channel if a particle is detected in the vicinity of the inlet of the channel by the monitoring device. This monitoring of the vicinity of the inlet of the channel ensures that only one single particle is aspirated through the inlet.

According to an embodiment, the device comprises a monitoring device or the monitoring device described above. The monitoring device is configured to obtain information about a size and shape of the single particle, i.e., of the single particle in the vicinity of the inlet to be aspirated through the inlet, and information about a time instant at which the single particle is aspirated through the inlet. Additionally, the device comprises a calculator configured to determine a position of the single particle, i.e., the aspirated particle, within the channel based on the information obtained by the monitoring device, i.e. the information about the size and shape of the single particle and the information about the time instant at which the single particle is aspirated through the inlet, and based on an information about a flow velocity through the channel. The flow velocity, for example, is given or controlled by the pressure generator. Alternatively, it is also possible to obtain the flow velocity from a channel monitoring device configured to monitor a predetermined volume of the channel and determine the flow velocity. This enables a tracking of the single particle within the channel and a controlled transport of single particles to their destined position.

According to an embodiment, the device comprises a decision-based ejection device, e.g., a discarding device, configured to determine, based on the position of the single particle within the channel obtained from the calculator, information on whether the single particle, i.e., the aspirated single particle, is present in a predetermined volume of the channel located near the outlet of the channel. The information indicates whether the single particle is in a vicinity of the outlet. The predetermined volume may indicate a liquid volume in the channel to be output through the outlet with the next ejection by the pressure generator. The calculator, for example, is configured to determine the position of the single particle at different time instances to track the transport of the particle through the channel. The decision-based ejection device, for example, is configured to determine, based on the position of the single particle within the channel obtained from the calculator, a time instant at which the single particle is in the predetermined volume of the channel. The decision-based ejection device is configured to eject liquid without the single particle to a first target position and liquid with the single particle to a second target position. The decision whether the liquid in the predetermined volume is ejected to the first target position or to the second target position depends on whether the single particle is in the liquid in the predetermined volume or not. For example, the decision-based ejection device is configured to eject liquid to the first target position until liquid including the single particle is to be output through the outlet. Then the decision-based ejection device is configured to eject the liquid including the single particle to the second target position. This allows to precisely transport an individual particle to a target position and reduces the possibility of ejecting liquid with or without a particles incorrectly, i.e. to the wrong target position.

According to an embodiment, the device comprises a channel volume monitoring device, i.e. a channel monitoring device, configured to monitor a predetermined volume of the channel located near the outlet of the channel and obtain information on whether the single particle, e.g., the aspirated single particle, is present in the predetermined volume. The predetermined volume might be defined as described above. The decision-based ejection device is configured to eject liquid without the single particle to a first target position, if the information on whether the single particle is present in the predetermined volume indicates that the single particle is not present in the liquid to be output, and eject liquid with the single particle to a second target position, if the information on whether the single particle is present in the predetermined volume indicates that the single particle is present in the liquid to be output. The liquid to be output, for example, is the liquid within the predetermined volume of the channel. The channel volume monitoring device reduces the possibility of ejecting liquid with or without a particles incorrectly, i.e. to the wrong target position. The device may comprise the channel volume monitoring device additionally to the above described calculator for determining the position of the single particle within the channel. The two systems can work together to even more improve an accuracy at an ejection of liquid to its target position.

According to an embodiment, the device comprises a particle concentration monitoring device configured to determine a particle concentration in the liquid including the particles in the reservoir and to indicate whether the particle concentration exceeds a predetermined threshold, e.g., a predetermined concentration threshold. A high concentration of particles increases the probability that not only a single particle is aspirated. Therefore, the particle concentration monitoring device reduces the probability of aspirating two or more particles instead of only one. If the particle concentration monitoring device indicates that the particle concentration exceeds a predetermined threshold, a person using the device can adapt the concentration so that the concentration is below the predetermined threshold. Alternatively, the device can further comprise a concentration adaptation device configured to reduce a concentration of particles in the liquid in the reservoir, if the particle concentration monitoring device indicates that the particle concentration exceeds a predetermined threshold. For example, the concentration adaptation device may be configured to add liquid, i.e. liquid without particles, to the reservoir to reduce the particle concentration. The predetermined threshold may be equal to 50 particle/ml, 100 particle/ml 500 particle/ml, 1000 particle/ml, 5000 particle/ml or 10 000 particle/ml. The predetermined threshold, e.g., a predetermined particle concentration threshold, may depend on the size and/ or geometry of the reservoir.

According to an embodiment, the channel is a continuous filter free channel. The device is configured to transport specific single particles, for which reason it is not necessary to integrate filter into the channel. Additionally, it is advantageous that the channel is a continuous channel, since such a channel is easy to clean/sterilize. Even if some particles would sediment within the channel, such sedimented particles can be removed easily from the continuous filter free channel, since no hindering structures are integrated in the channel.

According to an embodiment, the channel is a, e.g., disposable, hose or tube with a uniform diameter over its complete length. Thus the device is easy to implement and can easily satisfy hygienic standards, since the hose or tube can be easily replaced by a new one. Even if some particles would sediment within the channel, such sedimented particles can be removed easily from the channel, because of its uniform diameter.

According to an embodiment, the pressure generator is configured to output the single particle through the outlet of the channel by dispensing the single particle in a free-flying droplet. For example, the pressure generator comprises a pressure actuator configured to press against the channel and deform same to eject liquid including the single particle in a droplet. The pressure actuator is configured to release the channel after pressing against same to generate the pressure gradient, i.e., a pressure gradient in the direction of the outlet. The channel may be out of a deformable material, like a polymer material.

A further embodiment relates to a method for particle isolation comprising the steps fluidically coupling an inlet of a channel to a reservoir holding a liquid including particles and causing a relative movement between the particles and the inlet of the channel so as to bring a single particle in a vicinity of the inlet to be aspirated through the inlet. Additionally, the method comprises generating a pressure gradient in the channel by which liquid including the single particle is aspirated from the reservoir through the inlet of the channel against the force of gravity and outputting liquid through an outlet of the channel.

The method as described above is based on the same considerations as the above-described device. The method can, by the way, be completed with all features and functionalities, which are also described with regard to the device, wherein the features and/or functionalities correspond to features of a method step and/or a method step.

### Brief Description of the Drawings

The drawings are not necessarily to scale, emphasis instead generally being placed upon illustrating the principles of the invention. In the following description, various embodiments of the invention are described with reference to the following drawings, in which:
- Fig. 1: shows an embodiment of a device for particle isolation;
- Fig. 2: shows a more detailed embodiment of the device for particle isolation;
- Fig. 3: shows an actuator configured to move the reservoir and/or the inlet of the channel;
- Fig. 4: shows a microchip connected to an inlet of the channel of the device;
- Fig. 5: shows an actuator configured to induce mixing liquid flows in the reservoir using a pump;
- Fig. 6: shows an actuator configured to induce mixing liquid flows in the reservoir using a magnetic principle;
- Fig. 7: shows an embodiment of a droplet-generating-device as example for the pressure generator;
- Fig. 8: shows a pump as example for the pressure generator;
- Fig. 9: shows an embodiment comprising a collection chamber with functional structures; and
- Fig. 10: shows a block diagram of a method for isolating particles.

### Detailed Description of the Embodiments

Equal or equivalent elements or elements with equal or equivalent functionality are denoted in the following description by equal or equivalent reference numerals even if occurring in different figures.

In the following description, a plurality of details is set forth to provide a more throughout explanation of embodiments of the present invention. However, it will be apparent to those skilled in the art that embodiments of the present invention may be practiced without these specific details. In other instances, well-known structures and devices are shown in block diagram form rather than in detail in order to avoid obscuring embodiments of the present invention. In addition, features of the different embodiments described herein after may be combined with each other, unless specifically noted otherwise.

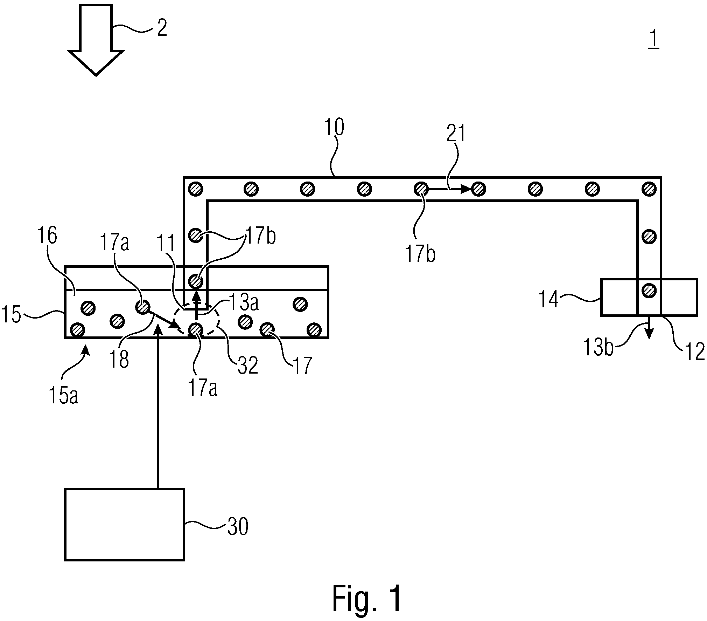

Fig. 1 shows an embodiment of a device 1 for particle isolation. The device 1 comprises a reservoir 15 to contain a liquid 16 including particles 17. The particles 17 have the same or a higher density than the liquid 16 in the reservoir 15. Additionally, the device 1 comprises a channel 10 with an inlet 11 and an outlet 12. The channel 10 might be a continuous filter free channel. The channel may be a hose with a uniform diameter over its complete length.

The inlet 11 of the channel 10 is configured to be fluidically coupled to the reservoir 15. For example, a part of the channel 10 comprising the inlet 11 can be hung into the reservoir. As shown in Fig. 1, the channel 10 may extend into the reservoir 15 so as to fluidically couple the inlet with the reservoir. For example, a supporting structure may be configured to hold the part of the channel 10 comprising the inlet 11 at a stationary position, wherein the part of the channel 10 comprising the inlet 11 extends into the reservoir 15. Alternatively, for example, an actuator, like a robot or a stage, may be configured to move the part of the channel 10 comprising the inlet 11 so as to fluidically couple the inlet 11 to the reservoir 15. The part of the channel 10 comprising the inlet 11 may be arranged in a stationary manner or in a movable manner.

Additionally, the device 1 comprises a pressure generator 14 configured to generate a pressure gradient in the channel 10 by which liquid including a single particle 17a is aspirated 13a from the reservoir 15 through the inlet 11 of the channel 10 against the force 2 of gravity. The aspiration against the force 2 of gravity has the effect of reducing the probability of aspirating a plurality of particles clogging the channel. The aspiration against the force 2 of gravity increases the probability of aspirating only one particle, e.g., per time instant. The single particle 17a represents one of the particles 17 within the liquid 16 in the reservoir 15. A pressure-gradient force resulting from the pressure gradient acts on the single particle 17a so as to aspirate the single particle 17a. A direction of the pressure-gradient force is directed from the inlet 11 of the channel 10 to the outlet 12 of the channel 10 along the channel 10. According to the pressure-gradient a low pressure is realized in a part of the channel 10 comprising the outlet 12 and a high pressure is realized in the part of the channel 10 comprising the inlet 11. The pressure-gradient force is directed from the region of higher-pressure to the region of lower-pressure, i.e. from the inlet 11 to the outlet 12.

As shown in Fig. 1, the part of the channel 10 comprising the inlet 11 is arranged in an angle of 90° with respect to a bottom surface 15a of the reservoir 15. Thus the single particle 17a has to be aspirated 13a against the force 2 of gravity. Another angle would also be possible, wherein the inventors found that an angle of at least 10°, 20° or 30° achieves a good aspiration 13a of individual particles 17. This is based on the idea that such an angle enables a controlled, active and specific aspiration of single particles. Such an angle enables an easy implementation of the relative movement caused by the actuator 30, e.g., especially of the relative movement between the reservoir 15 and the inlet 11. Furthermore such an angle decreases a probability of additionally aspirating neighboring particles 17, i.e. particles 17 neighboring the single particle 17a in the reservoir 15. Especially advantageous with respect to a probability of aspirating 13a only one single particle 17a through the inlet 11 and with respect to an efficiency of a transport of the single particle 17a through the channel 10 is an angle of 90°. An angle *α* in the range of 10° ≤ *α* ≤ 90° might be especially advantageous.

As shown in Fig. 1, the single particle 17a aspirated 13a by the pressure generator 14 through the inlet 11 is transported through the channel 10. In the following, the single particle 17a in the channel 10 may be understood as an aspirated particle 17b. The pressure gradient in the channel 10 induces a flow 21 transporting the aspirated particle 17b to the outlet 12 of the channel 10.

Additionally, the pressure generator 14 is configured to output 13b liquid 16 with or without the single particle 17a, i.e. the aspirated particle 17b, through the outlet 12 of the channel 10. The pressure generator 14 may be configured to output 13b a predetermined volume of liquid 16 through the outlet 12 at a certain time instant. The pressure generator 14 may be configured to first output 13b a predetermined volume of liquid 16 with or without a single particle 17a through the outlet 12 and then aspirate 13a the same amount of liquid with or without a single particle 17a through the inlet 11. Thus, the outputting 13b and the aspiration 13a of the pressure generator 14 work together to achieve an efficient transport of single particles 17a from the reservoir 15 to a target position. The outputting 13b and the aspiration 13a may be performed by the pressure generator 14 on demand.

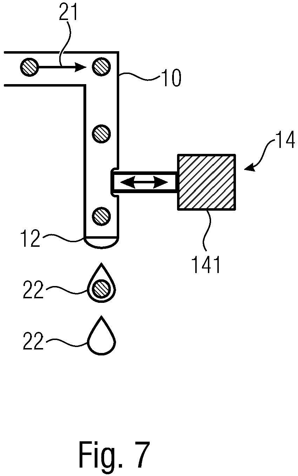

The pressure generator 14 may be implemented as described with regard to one of Figs. 7 to 9.

Furthermore, the device 1 comprises an actuator 30 configured to cause a relative movement 18 between the particles 17 and the inlet 11 of the channel 10 so as to bring the single particle 17a in a vicinity 32 of the inlet 11 to be aspirated 13a through the inlet 11. In order to achieve the relative movement the channel 10, the reservoir 15 and/or the particle 17a itself may be moved by the actuator 30. The actuator 30 might comprise one or more of
- a motorized positioner for moving the channel 10 in z-dimension, in xy-dimensions or in xyz-dimensions;
- a motorized positioner for moving the reservoir in z-dimension, in xy-dimensions or in xyz-dimensions;
- a vibration device for moving the reservoir; and
- a flow generation device for inducing microfluidic flows or mixing flows in the reservoir 15.

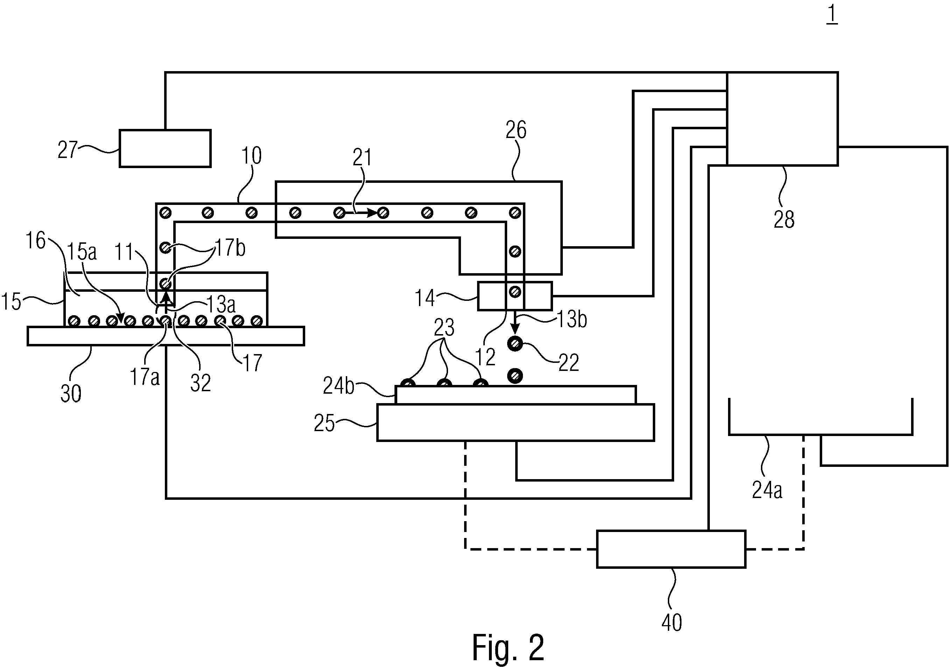

Different types of actuators, which can be implemented individually or in combination in the device in Fig. 1, are described with regard to Figs. 2 to 6. For example, a flow generation device, a vibration device or a motorized positioner for moving the reservoir 15 may be combined with a motorized positioner for moving the channel 10.

The pressure generator 14 may be configured to generate the pressure gradient in the channel 10 on demand, e.g., at time instances at which a single particle 17a is in the vicinity 32 of the inlet 11. For example, the device 1 may comprise a monitoring device configured to monitor the vicinity 32 of the inlet 11 and trigger the pressure generator 14 at a time instant at which a single particle 17a is detected in the vicinity 32 of the inlet 32. The pressure generator 14 may be configured to generate the pressure gradient in response to the trigger received from the monitoring device.

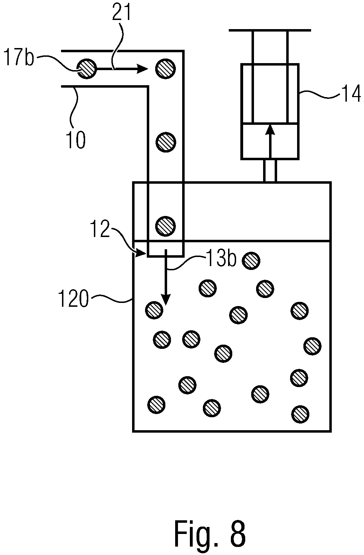

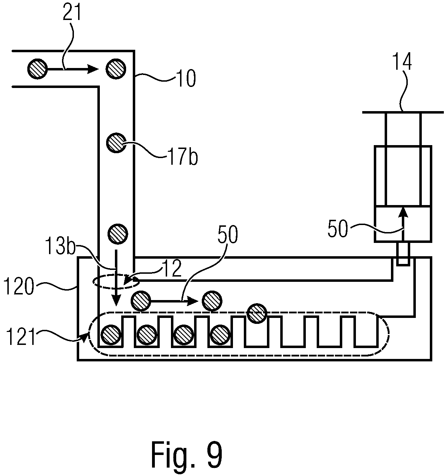

As shown in Fig. 1, the pressure generator 14 may be arranged at the outlet 12 of the channel 10. Alternatively, it is also possible that the pressure generator 14 is arranged near the outlet 12 of the channel 10, e.g., the outlet 12 of the channel 10 may be connected to a chamber into which the liquid 16 with or without the single particle 17a is to be output by the pressure generator 14 and the pressure generator 14 may be connected to the chamber to generate the pressure gradient in the channel 10 over the chamber, for example, see Figs. 8 and 9.

The invention is a concept for isolating particles 17, e.g., cells, spheroids or tumoroids, in a liquid medium 16 in a reservoir 15 by aspirating 13a the particles 17 individually from the reservoir 15, guiding them through a channel 10, like a thin tube or capillary, and transporting them to a target position. The device 1, for example, provides an automated platform for in vitro experiments with 3D cell culture models (spheroids, organoids, e.g., microtumors), which can be used in the clinical environment initially for the purpose of personalized therapy selection.

The device 1 is configured to isolate particles 17 present in a liquid medium 16 by an aspiration process 13a and transport the isolated particle, i.e. the single particle 17a, through a channel 10, like a disposable tube, and to direct them to the tube output 12. The device 1 describes a liquid handling platform comprising: the channel 10, e.g., a disposable tube, acting as an aspirating opening 11 at one end and as an output 12 at the other end, to which a suction may be applied that is generated by the pressure generator 14, i.e. a suction generating device, and that is directed in the output direction. The aspirating opening, i.e. the inlet 11, may be immersed in the reservoir 15, which is configured to contain the liquid medium 16 and particles 17. Particles 17 are moved into the vicinity 32 of the aspirating opening 11 by a device, i.e. the actuator 30. The actuator 30 may be arranged in or at the reservoir 15 to generate particle movement, i.e. the relative movement 18. Particles 17a in the vicinity 32 of the aspirating opening 11 may be aspirated from the aspirating opening 11 into the tube 10 by suction applied to the tube 10. Aspirated particles 17b may be transported through the tube 10 to the output, i.e. the outlet 12, by a microfluidic flow 21 generated by the pressure generator 14.

In other words, Fig.1 shows a device 1 for isolated absorption of particles 17 in a liquid medium 16 from a reservoir 15. The device 1 comprises a channel 10, like a disposable tube, having two openings, one opening serving as an aspirating input, i.e. the inlet 11, and the other opening serving as an output, i.e. the outlet 12. Additionally, the device 1 comprises a reservoir 15 to contain particles 17 and liquid medium 16, in which the aspirating opening, i.e. the inlet 11, of the channel 10 may be located. Furthermore, the device may comprises a pressure generator 14, like a suction generating device, at the channel 10 that generates, for example, a suction on demand that aspirates a single particle 17a in the vicinity 32 of the aspirating opening 11 into the channel 10. Additionally, the device 1 comprises a moving device, i.e. the actuator 30, configured to move particles 17 into the vicinity 32 of the aspirating opening 11 of the channel 10.

The device 1 can comprise features and/or functionalities as described with regard to Figs. 2 to 9.

Fig. 2 shows optional additional features and/or functionalities for the device 1 of Fig. 1. The device 1 of Fig. 1 may comprise one or more of the features and/or functionalities described in the following:

According to an embodiment, the reservoir 15 is mounted on a motor-operated reservoir table. The motor-operated reservoir table represents the actuator 30 and is configured to move the reservoir 15 so as to bring a single particle 17a into the vicinity 32 of the inlet 11 of the channel 10. As shown in Fig. 2, the particles 17 may be sedimented on a bottom surface 15a of the reservoir 15 and the actuator 30 may be configured to move the reservoir 15 without disturbing the sedimented particles 17, e.g., using a velocity equal to or smaller than 1 mm/s, 5 mm/s, 10 mm/s or 20 mm/s and an acceleration equal to or smaller than 3 mm/s², 25 mm/s², 50 mm/s² or 100 mm/s² for moving the reservoir 15. Thus, the particles 17 have a fixed position within the reservoir 15 and the actuator 30 is configured to move systematically the reservoir 15 in three dimensions so as to move the particles 17 together with the reservoir 15 relative to the inlet 11 of the channel 10.

According to an embodiment, the device 1 comprises a reservoir monitoring device 27, i.e. a monitoring device, configured to monitor the reservoir 15 or at least a monitoring area of the reservoir 15 to obtain information about a position of the single particle 17a in the reservoir 15. The actuator 30 is configured to cause the relative movement to bring the single particle 17a in the vicinity 32 of the inlet 11 based on the information about the position of the single particle 17a in the reservoir 15. For example, the actuator 30 is configured to move the reservoir 15 and with it also the sedimented particle 17a relative to the inlet 11 so that the position of the single particle 17a lies within the vicinity 32 of the inlet 11. The actuator 30 may be configured to obtain the information about the position of the particle 17a directly from the reservoir monitoring device 27 and determine the necessary movement of the reservoir 15 so as to bring the single particle 17a to the inlet 11. Alternatively, the device 1 may comprise a processor 28 configured to obtain the information about the position of the single particle 17a in the reservoir 15, determine an information about a necessary movement of the reservoir 15, and provide the information about the necessary movement of the reservoir 15 to the actuator 30 so that the actuator 30 can apply the necessary movement to the reservoir 15.

According to an embodiment, the reservoir monitoring device 27 is configured to obtain information about positions of particles 17 within the monitoring area of the reservoir monitoring device 27. The processor 28 may be configured to determine distances between the particles 17 positioned in the monitoring area based on the positions of the particles 17 provided by the reservoir monitoring device 27. The processor 28 may be configured to consider the determined distances to select the single particle 17a to be aspirated through the inlet 11. For example, the processor may be configured to check for each particle 17 within the monitoring area whether all neighboring particles have at least a predetermined distance to the respective particle 12. If this is the case for a certain particle 17, the processor 28 may be configured to select same as the single particle 17a to be aspirated 13a. The processor 28 is configured to provide the information about the position of the selected single particle 17a to the actuator 30.

A combination of the reservoir monitoring device 27, e.g., for the reservoir volume, and the motor-operated reservoir table, i.e. the actuator 30, enables to selectively move sedimented particles 17 in the reservoir 15 to the vicinity 32 of the aspirating opening, i.e. the inlet 11.

According to an embodiment, the reservoir monitoring device 27 or another monitoring device can be configured to monitor the vicinity 32 of the inlet of the channel 10 to detect particles 17. In case of detecting a single particle 17a in the vicinity of the inlet 11, the reservoir monitoring device 27 or the other monitoring device may be configured to trigger the pressure generator 14 and the pressure generator 14 is configured to generate the pressure gradient in the channel 10 in response to the received trigger. Alternatively, the controller/processor 28 may be configured to control the pressure generator 14 to cause the pressure gradient in the channel 10 if a particle 17a is detected in the vicinity 32 of the inlet 11 of the channel 10 by the reservoir monitoring device 27 or the other monitoring device.

According to an embodiment, the reservoir monitoring device 27 or another monitoring device, like the one described above, can be configured to obtain information about properties, like size, material and/or shape, of the single particle 17a and information about a time instant at which the single particle 17a is aspirated through the inlet 11. A calculator, e.g., the processor 28, may be configured to determine a position of the single particle 17a within the channel 10 based on
- the information about the size of the single particle 17a,
- the information about the material of the particle 17a,
- the information about the shape of the particle 17a,
- the information about the time instant at which the single particle 17a is aspirated through the inlet 11, and
- an information about a flow rate through the channel 10.

In other words, the particle position in the channel 10 can be calculated based on the aspiration time, the tube flow 21 and the particle properties, so that it can be determined at what time aspirated particles 17b are located at a certain position in the channel 10. The device 1 may comprise a decision-based ejection device 40 configured to determine, based on the position of the single particle 17a within the channel 10 whether the single particle 17a is present in a predetermined volume of the channel 10 located near the outlet of the channel. The predetermined volume, for example, represents a volume of the liquid 16 to be output next by the pressure generator 14. The decision-based ejection device 40 may be configured to eject the liquid within the predetermined volume to a first target position 24b, if the single particle 17a is not present in the predetermined volume, and to a second target position, if the single particle 17a is present in the predetermined volume.

According to an embodiment, the device 1 comprises a channel volume monitoring device 26 configured to monitor a predetermined volume of the channel 10 located near the outlet 12 of the channel 10. The channel volume monitoring device 26 is configured to obtain information on whether the single particle 17a will be present in the liquid 16 to be output at the next outputting performed by the pressure generator 14, e.g., information on whether the single particle 17a will be present in the next droplet 22 output through the outlet 12. Optionally, the predetermined volume represents the volume of the liquid 16 to be output next. In this case, the information whether the single particle 17a is present in the predetermined volume indicates directly whether the single particle 17a will be present in the liquid 16 to be output at the next outputting performed by the pressure generator 14. Alternatively, the predetermined volume may represent a volume of the channel 10 comprising the volume of the liquid 16 to be output next or may represent a volume of the channel 10 arranged before the volume of the liquid 16 to be output next, as shown in Fig. 2. In this case, the channel volume monitoring device 26 or the processor 28 may be configured to determine a position of the single particle 17a within the predetermined volume at a certain time instance and calculate the time instance at which the single particle 17a will be present in the volume of the liquid 16 to be output by the pressure generator 14. For example, the channel volume monitoring device 26 may provide information on the next dispensed droplet 22, e.g., information about the presence or absence of a particle in the droplet 22.

According to an embodiment, the device 1 may additionally to the channel volume monitoring device 26 comprise the decision-based ejection device 40 configured to eject liquid 16 with the single particle 17a to a second target position 24b, like a collecting substrate, if the channel volume monitoring device 26 or the processor 28 indicates that the single particle is present in the liquid to be output. Otherwise, the decision-based ejection device 40 may be configured to eject liquid 16 without the single particle 17a to a first target position 24a, i.e. if the information indicates that the single particle 17a is not present in the liquid 16 to be output. The first target position 24a might also be understood as a reject volume or discarding volume. The decision-based ejection device 40 may be configured to either move a part of the channel 10 comprising the outlet 12 or move the first target position 24a and the second target position 24b so as to eject the liquid 16 to be output by the pressure generator 14 to the correct target position 24a or 24b. As shown in Fig. 2, it is possible that the decision-based ejection device 40 is configured to eject the liquid 16 with the single particle 17a to a certain position within the second target position 24b. The decision-based ejection device 40 may be configured to separate and sort droplets 22 with and without a particle using vacuum extraction, a motor-operated stage, an electromagnetic deflection device, a pneumatic deflection device or an electric deflection device.

For example, the channel volume monitoring device 26 provides information on the predetermined volume of the channel 10, like a flow velocity within the predetermined volume and/or positions of particles 17 within the predetermined volume, and/or whether a single particle 17a dispensed in a droplet 22 will be output by the pressure generator 14. A deposition location 23 of the dispensed droplets 22 can be approached by a motor-operated target substrate table 25. The decision-based ejection device 40 may be configured to control the motor-operated target substrate table 25 to eject the droplet 22 to the second target position 24b, e.g., to a deposition location 23 within the second target position 24b.

According to an embodiment, the device 1 can comprise the channel volume monitoring device 26 and the above described calculator for calculating a position of a single particle 17a within the channel 10 to improve a decision of the decision-based ejection device 40 whether liquid output through the outlet 12 has to be ejected to the first target position 24a or the second target position 24b.

Alternatively, the device 1 can comprise a droplet observation device for acquiring information on dispensed droplets 22, e.g., information on the content of the dispensed droplets 22. The information on the drop 22 may indicate at least the presence or absence of a particle in the drop volume. The decision-based ejection device 40 may be configured to move the resulting droplets 22 to a reject position, i.e. the first target position 24a, with a motor-operated table, pneumatic or electric deflection device, in the absence of particles in the droplet 22.

The herein described monitoring devices, like the reservoir monitoring device 27 and the channel volume monitoring device 26, can represent optical observation devices, such as camera or light absorption detectors, or impedance sensors to observe the reservoir volume or the tube volume, i.e. a predetermined channel volume. The reservoir monitoring device 27 may be located outside the reservoir 15 and the channel volume monitoring device 26 may be located outside the channel 10.

The reservoir monitoring device 27 may be configured to obtain information on position, number, size and shape of particles 17 and on whether and at what time a particle 17 is aspirated.

According to an embodiment, the device comprises a controller/processor 28. Thus the system is controlled by a processor 28 and automated. This results in an efficient transport of the particles 17.

According to an embodiment, the pressure generator 14 represents a droplet generating device. The pressure generator 14 is configured to output liquid 16 in the form of a droplet 22, e.g., the pressure generator 14 may be configured to output the single particle 17a through the outlet 12 of the channel 10 by dispensing the single particle 17a in a free-flying droplet 22. A suction may be generated by the droplet generating device, e.g., to aspirate liquid 16 with or without a single particle 17a through the inlet 11.

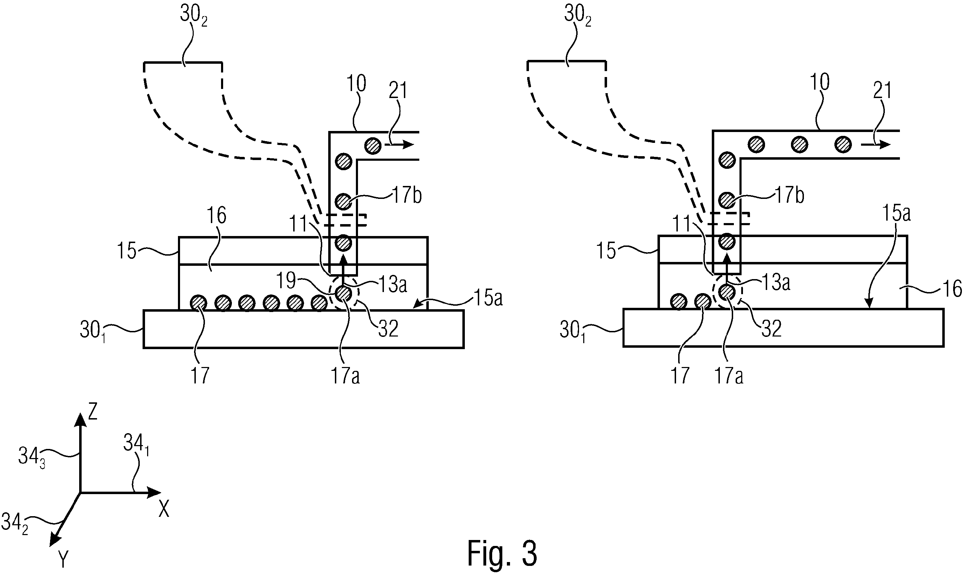

Fig. 3 shows exemplarily an aspiration process 13a of a herein described device 1. The actuator 30, i.e. 30₁ and/or 30₂, is configured to move the particles 17 relative to the inlet 11 of the channel 10 so as to bring a single particle 17a in the vicinity 32 of the inlet 11 to be aspirated 13a through the inlet 11.

According to a first embodiment, the actuator 30 represents a motor-operated reservoir positioner, i.e. a motorized positioner, 30₁ configured to move the reservoir 15 relative to a stationary inlet 11 of the channel 10. The movement may be performed in two dimensions or in all three dimensions.

According to a second embodiment, the actuator 30 represents a motor-operated channel positioner, i.e. a motorized positioner, 30₂ configured to move the channel 10 relative to a stationary reservoir 15. The movement may be performed in two dimensions or in all three dimensions.

According to a third and preferred embodiment, the actuator 30 comprises a first actuator, e.g., the motor-operated reservoir positioner 30₁, and a second actuator, e.g., the motor-operated channel positioner 30₂, to perform the relative movement between the single particle 17a in the reservoir 15 and the inlet 11 of the channel 10. The first actuator 30₁ may be configured to move the reservoir 15 in an x-dimension 34₁ and a y-dimension 34₂ and the second actuator 30₂ may be configured to move the inlet 11 of the channel in a z-dimension 34₃.

In one embodiment of the invention, the reservoir 15 comprises a bottom surface 15a, which is large enough so that the particles 17 contained in the liquid medium 16 can sediment in a monolayer on the bottom surface 15a. The actuator 30 is configured to perform the relative movement so that the sedimented particles 17 can be aspirated through the inlet 11.

The particles 17 can either specifically or randomly be delivered to the vicinity 32 of the aspirating opening 11 by the actuator 30. The specific delivery may be achieved together with the aforementioned reservoir monitoring device 27 providing an information of a position of a single particle 17a. With the knowledge of the position of the single particle 17a in the reservoir 15, the actuator 30 is configured to perform a specific relative movement to bring the single particle 17a into the vicinity of the inlet 11.

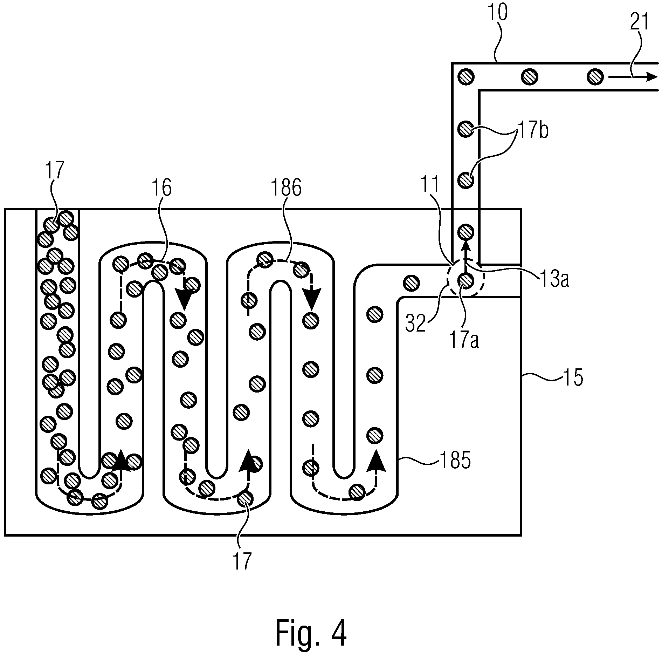

In one embodiment of the invention, a microfluidic chip is used as the reservoir 15 of device 1, see Fig. 4. Particles 17 are focused by the special geometry of flow channels 185 and/or by microfluidic flows 186 so that they are arranged at regular distances from each other. Particles 17 may be transported to the inlet 11 at regular intervals. The actuator 30 of device 1 is configured to induce a flow 186 in the microfluidic chip to separate the particles 17 from each other and to transport the particles 17 individually into the vicinity 32 of the inlet 11 of the channel 10. The actuator 30 may represent a pump inducing the flow 186 in the microfluidic chip. The separation of the particles 17 may be assisted by a meander-shaped microchannel, e.g., a reservoir channel, in the microfluidic chip. The meander-shaped microchannel in the microfluidic chip can contain the liquid 16 and the particles 17. As shown in Fig. 4, an end portion of the microchannel is linked, i.e. fluidically coupled, to the inlet 11 of the channel 10 to enable an aspiration 13a of a single particle 17a from the reservoir 15 through the inlet 11 into the channel 10. A microfluidic chip may contain chambers and/or channels, i.e. microchannels, through which fluids flow, wherein the chambers and/or channels have dimension in the micrometer range or below. A microfluidic chip may comprise a pattern of microchannels, molded or engraved.

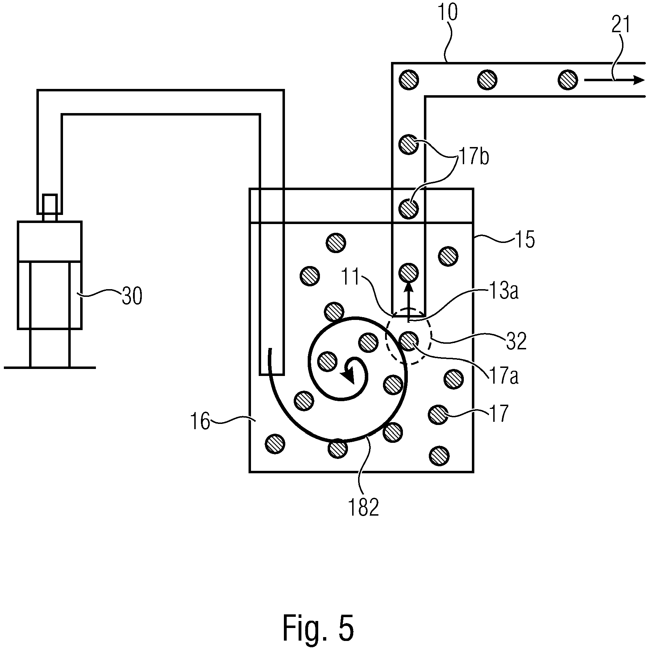

Fig. 5 shows that the actuator 30 of device 1 can represent a pump configured to induce a flow 182 in the reservoir 15. The particles 17, for example, are in suspension in the reservoir 15. The actuator 30 is configured to transport, e.g., randomly, the particles 17 separately to the inlet 11. A random particle movement can be induced, for example, by a pump, like a syringe pump, roller pump, etc. In Fig. 5, the pump is configured to cause mixed flows 182 in the reservoir 15. For example, the actuator 30 is configured to induce the flow 182 to lift sedimenting particles 17 to the inlet 11 immersing into the reservoir 15. The actuator 30 may be configured to induce the flow 182 to separate the particles 17 within the reservoir, e.g., the flow 182 may prevent an aggregation or accumulating of the particles 17, especially in the vicinity 32 of the inlet 11.

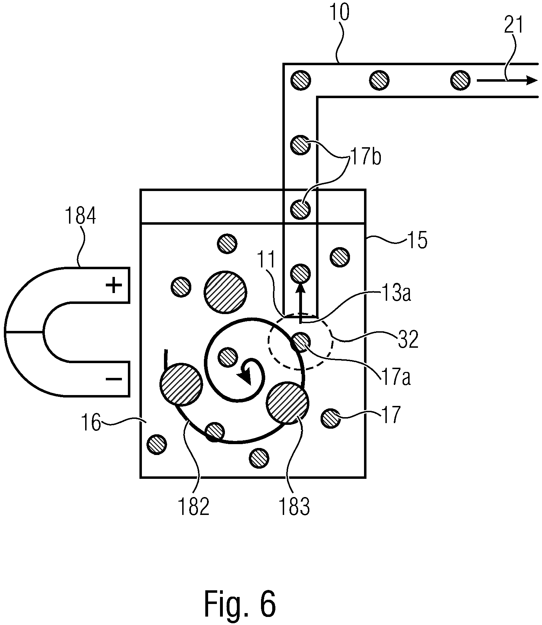

Likewise, mixed flows 182 can be induced in the reservoir 15 by paramagnetic particles 183. The actuator 30 of device 1 may comprise a permanent magnet 184 arranged outside the reservoir 15 and paramagnetic particles 183 arranged inside the reservoir 15. The actuator 30 is configured to induce stirring movements of the paramagnetic particles 183 inside the liquid 16 in the reservoir 15 by the permanent magnet 184, see Fig. 6. The flow 182 induced by the actuator 30 lifts or transports the particles 17 to the inlet 11, e.g., as described with regard to the flow 182 in Fig. 5.

In case the device 1 comprises one of the actuators 30 described with regard to Fig. 5 and Fig. 6, the device 1 may additionally comprise a particle concentration monitoring device configured to determine a particle concentration in the liquid 16 including the particles 17 in the reservoir 15. A particle concentration equal to or below a predetermined threshold increases a probability of aspirating single particles 17 through the inlet. A particle concentration higher than the predetermined threshold increases a probability of aspirating more than one particle 17 at the same time or rather allows an aspiration of two or more particles close together. Therefore, it is desired that the particle concentration in the reservoir 15 is below the predetermined threshold so that single particles are aspirated in certain time intervals. For example, the pressure generator 14 may be configured to output a certain amount of liquid 16 per time instant and the predetermined threshold defines a particle concentration at which only one particle 17 is present in the certain amount of liquid 16 output by the pressure generator 14 per time instant. The particle concentration monitoring device may be configured to indicate whether the particle concentration exceeds the predetermined threshold or may be configured to automatically adapt the particle concentration by adding further liquid 16 to the reservoir 15 to reduce the particle concentration, if the particle concentration exceeds the predetermined threshold.

In one embodiment of the invention, a suction applied to the tube output, i.e. the outlet 12, may be generated by a droplet generating device, as shown in Fig. 7. The droplet generating device may represent the pressure generator 14 of the herein described device 1. For example, a piezo-driven plunger 141 can be used that dispenses droplets 22 from the tube output 12 by actuation on the tube, i.e. the channel 10. The device 1 may comprise a flexible or deformable channel 1. The plunger 141 of the pressure generator 14 is configured to deform the channel 10 near the outlet 12 to output a certain amount of liquid 16 with or without a single particle 17a, e.g., in the form of a droplet 22. Furthermore, the plunger 141 of the pressure generator 14 is configured to stop deforming the channel 10 near the outlet 12 to induce a pressure gradient within the channel 10 and aspirate liquid 16 with or without a single particle 17a through the inlet 11 of the channel 10. Alternatively, a thermal droplet generating device may be used as the pressure generator 14.

Figures 8 an 9 show further options for the pressure generator 14 of the herein described device 1. Both embodiments have in common that the outlet 12 and the pressure generator 14 are in connection with a collection chamber 120. The pressure generator 14 may be configured to create a negative pressure in the collection chamber 120 and therefore induce a pressure gradient within the channel 10 for the aspiration process 13a through the inlet 11 of the channel 10, for the transport 21 through the channel 10 and for the outputting 13b through the outlet 12 of the channel 10. A suction 50 may be generated by a negative or positive pressure induced by the pressure generator 14. The pressure generator 14 in Figs. 8 and 9 may be realized as a pump, like a syringe pump, roller pump, etc. For example, the pump can be configured to generate in a collection vessel, i.e. the collection chamber120, a negative pressure and thus the suction 50 that transports aspirated particles 17b into the collection vessel 120. This type of configuration of the invention can be used to very easily and quickly discharge a clearly defined number of particles 17 from a particle suspension.

The embodiments shown in Figs 8 and 9 differ in that, the collection vessel 120 in Fig. 9 further comprises functional structures 121 to further sort or manipulate the particles 17.

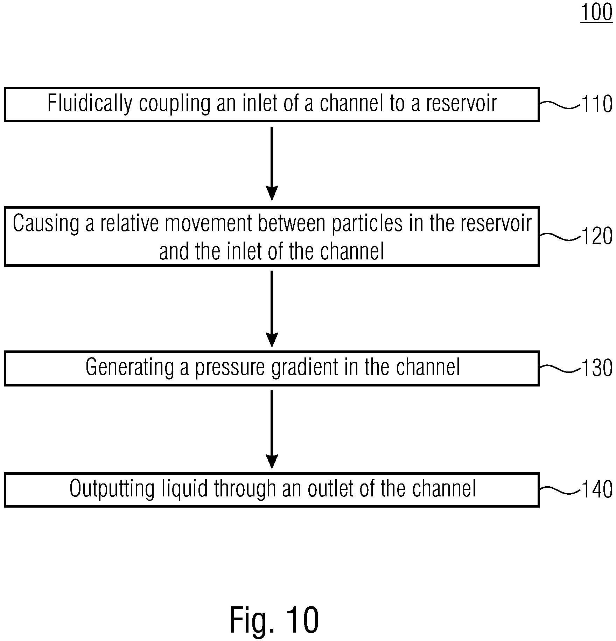

Fig. 10 shows an embodiment of a method 100 for isolating particles. The method 100 comprises fluidically coupling 110 an inlet of a channel to a reservoir holding a liquid including particles. Additionally, the method 100 comprises causing 120 a relative movement between the particles and the inlet of the channel so as to bring a single particle in a vicinity of the inlet to be aspirated through the inlet. Furthermore, the method 100 comprises generating 130 a pressure gradient in the channel by which liquid including the single particle is aspirated from the reservoir through the inlet of the channel against the force of gravity and outputting 140 liquid through an outlet of the channel.

According to an embodiment, the method 100 comprises causing 120 the relative movement by moving the reservoir and/or the inlet of the channel so as to bring the single particle, preferably a sedimented particle, in the vicinity of the inlet to be aspirated through the inlet. According to a preferred embodiment, the method 100 performs the causing 120 of the relative movement by moving the reservoir in two dimensions, and moving the channel in a third dimension perpendicular to a plane defined by the two dimensions.

According to an embodiment, the method 100 comprises causing 120 the relative movement by inducing mixing liquid flows in the reservoir to prevent particles from sedimenting in the reservoir and to bring the particles individually in the vicinity of the inlet of the channel.

According to an embodiment, the reservoir is a reservoir channel, e.g., a microchannel, within a microchip and the method 100 comprises fluidically coupling 110 the inlet to an end portion of the reservoir channel, and causing 120 the relative movement by causing a flow of the liquid in the reservoir channel to bring the particles individually in the vicinity of the inlet of the channel.

According to an embodiment, the method 100 comprises monitoring the reservoir to obtain information about a position of the single particles in the reservoir, and causing 120 the relative movement to bring the single particle in the vicinity of the inlet based on the information about the position of the single particle.

According to an embodiment, the method 100 comprises monitoring the vicinity of the inlet of the channel, and generating 130 the pressure gradient in the channel, if a particle is detected in the vicinity of the inlet of the channel.

According to an embodiment, the method 100 comprises obtaining information about a size and/or shape of the single particle and information about a time instant at which the single particle is aspirated through the inlet, and determining a position of the single particle within the channel based on the obtained information and based on an information about a flow velocity through the channel.

According to an embodiment, the method 100 comprises determining, based on the determined position of the single particle within the channel, information on whether the single particle is present in an end portion near the outlet of the channel, and ejecting liquid without the single particle to a first target position or with the single particle to a second target position based on this information.

According to an embodiment, the method 100 comprises monitoring a predetermined volume of the channel located near the outlet of the channel and obtaining information on whether the single particle is present in the predetermined volume. Additionally, the method 100 may comprise ejecting liquid without the single particle to a first target position, if the information indicates that the single particle is not present in the liquid to be output, and ejecting liquid with the single particle to a second target position, if the information indicates that the single particle is present in the liquid to be output.

According to an embodiment, the method 100 comprises determining a particle concentration in the liquid including the particles in the reservoir and indicating whether the particle concentration exceeds a predetermined threshold.

According to an embodiment, the method 100 comprises outputting 140 the single particle through the outlet of the channel by dispensing the single particle in a free-flying droplet.

An embodiment relates to a method 100 of selectively or randomly aspirating one or more particles passing through a disposable tube by the suction generating device, i.e. the pressure generator, comprising the steps of:
- filling a reservoir with a particle suspension into which the aspirating opening, i.e. the inlet, of a tube, i.e. the channel, is immersed.
- for selective aspiration (active): selecting a suitable particle with the reservoir monitoring device. Specifically moving the suitable particle to the vicinity of the aspirating opening of the channel, e.g., a disposable tube.
- for random aspiration (passive): moving the medium so that the particles are in suspension. Fluid movement brings particles randomly into the vicinity of the aspirating opening of the tube.
- acquiring information on the particles in the reservoir in the vicinity of the aspirating tube opening, i.e. the inlet, with the reservoir observation device, i.e. the reservoir monitoring device.
- generating a suction at the aspirating opening of the tube with the pressure generator, e.g., an on-demand suction generating device. Aspirating the particles in the vicinity of the aspirating opening into the tube.
- calculating the particle position in the tube using information collected by the reservoir monitoring device in dependence on the particle size, shape and material, aspiration time, and tube flow to determine when the aspirated particle reaches the tube output, i.e. the outlet.
- transporting the aspirated particle through the disposable tube by the flow generated with the suction generating device, i.e. the pressure generator.

Although some aspects have been described in the context of an apparatus, it is clear that these aspects also represent a description of the corresponding method, where a block or device corresponds to a method step or a feature of a method step. Analogously, aspects described in the context of a method step also represent a description of a corresponding block or item or feature of a corresponding apparatus.

The above described embodiments are merely illustrative for the principles of the present invention. It is understood that modifications and variations of the arrangements and the details described herein will be apparent to others skilled in the art. It is the intent, therefore, to be limited only by the scope of the impending patent claims and not by the specific details presented by way of description and explanation of the embodiments herein.

### Literature

Alberts, B.J. (2014). Molecular Biology of the Cell 6th ed. New York, NY: Garland Science.
Ayan, B.H. (2020). Aspiration-assisted bioprinting for precise positioning of biologics. Science advances
Bode, A. M. (2017). Precision oncology-the future of personalized cancer medicine?' S.1-2
Caponigro, G. (2011). Advances in the preclinical testing of cancer therapeutic hypotheses. Nature reviews drug discovery, S.179-187.
Cyfuse Biomedical K.K. (1. Marz 2019). Von https://www.cyfusebio.com/en/product/3dprinter/device/, Stand: 01.03.2019 abgerufen
Eglen, R. M. (2015). Drug discovery goes three-dimensional: goodbye to flat high-throughput screening? Assay and drug development technologies, S. 262-265.
Esch, E. W. (2015). Organs-on-chips at the frontiers of drug discovery. Nature reviews Drug discovery, S. 248-260.
Ghosh, R. N. (2011). Trastuzumab has preferential activity against breast cancers driven by HER2 homodimers. Cancer research, S. 1871-1882.
Gutzweiler, L. K. (2017). Large scale production and controlled deposition of single HUVEC spheroids for bioprinting applications. Biofabrication.
Lang, M. W. (2010). Integrated system for 3D assembly of bio-scaffolds and cells. IEEE International Conference on Automation Science and Engineering, S. 786-791.
Mark Howard, M. S. (2014). Australia Patentnr. AU2008232516 B2.
Peter Koltay, A. Y. (2010). Europe Patentnr. EP2577254A1.
Skardal, A. S. (2016). Organoid-on-a-chip and body-on-a-chip systems for drug screening and disease modeling. Drug discovery today, S. 1399-1411.
Stintzing, S. M. (2016). FOLFIRI plus cetuximab versus FOLFIRI plus bevacizumab for metastatic colorectal cancer (FIRE-3): a post-hoc analysis of tumour dynamics in the final RAS wild-type subgroup of this randomised open-label phase 3 trial. The Lancet Oncology, S. 1426-1434.
Viktor Shkolnikov, M. W. (2017). Taiwan Patentnr. TWI718425B.
Zhang, B. &. (2017). Organ-on-a-chip devices advance to market. Lab on a Chip, S. 2395-2420.

## Claims

1. Device (1) for particle isolation, comprising
a reservoir (15) to contain a liquid (16) including particles (17);
a channel (10) with an inlet (11) and an outlet (12), wherein the inlet (11) is configured to be fluidically coupled to the reservoir (15) containing the liquid (16) including particles (17);
a pressure generator (14) configured
to generate a pressure gradient in the channel (10) by which liquid (16) including a single particle (17a) is aspirated (13a) from the reservoir (15) through the inlet (11) of the channel (10) against the force (2) of gravity, and
to cause liquid (16) to be output through the outlet (12) of the channel (10);
an actuator (30) configured to cause a relative movement between the particles (17) and the inlet (11) of the channel (10) so as to bring a single particle (17a) in a vicinity (32) of the inlet (11) to be aspirated (13a) through the inlet (11).

2. Device (1) according to claim 1, wherein the actuator (30) is configured to move the reservoir (15) and/or the inlet (11) of the channel (10) so as to bring the single particle (17a), preferably a sedimented particle, in the vicinity (32) of the inlet (11) to be aspirated (13a) through the inlet (11).

3. Device (1) according to claim 1, wherein the actuator (30) is configured to induce mixing liquid flows (182) in the reservoir (15) to prevent particles (17) from sedimenting in the reservoir (15) and to bring the particles (17) individually in the vicinity (32) of the inlet (11) of the channel (10).

4. Device (1) according to claim 1, wherein the reservoir (15) is a reservoir channel (185) within a microchip and the inlet (11) is fluidically coupled to an end portion of the reservoir channel (185), wherein the actuator (30) is configured to cause a flow of the liquid (16) in the reservoir channel (185) to bring the particles (17) individually in the vicinity (32) of the inlet (11) of the channel (10).

5. Device (1) according to one of claims 1 to 4, wherein the device (1) comprises
a reservoir monitoring device (27) configured to monitor the reservoir (15) to obtain information about a position of the single particle (17a) in the reservoir (15), and
wherein the actuator (30) is configured to cause the relative movement to bring the single particle (17a) in the vicinity (32) of the inlet (11) based on the information about the position of the single particle (17a).

6. Device (1) according to claim 5,
wherein the reservoir monitoring device (27) is configured to obtain information about positions of particles (17) positioned in a monitoring area of the reservoir monitoring device (27);
wherein the device (1) further comprises a processor (28) configured
to determine distances between the particles (17) positioned in the monitoring area based on the information about the positions of the particles (17) positioned in the monitoring area,
to select from the particles (17) positioned in the monitoring area the single particle (17a) to be aspirated based on the determined distances between the particles (17), and
to provide the information about the position of the single particle (17a) to be aspirated to the actuator (30).

7. Device (1) according to one of claims 1 to 6, wherein the device (1) comprises
a monitoring device (27) configured to monitor the vicinity (32) of the inlet (11) of the channel (10), and
a controller (28) configured to control the pressure generator (14) to cause the pressure gradient in the channel (10) if a particle is detected in the vicinity (32) of the inlet (11) of the channel (10) by the monitoring device.

8. Device (1) according to one of claims 1 to 7, wherein the device (1) comprises
a monitoring device (27) or the monitoring device (27) configured to obtain information about a size and/or shape of the single particle (17a) and information about a time instant at which the single particle (17a) is aspirated (13a) through the inlet (11), and
a calculator (28) configured to determine a position of the single particle (17a) within the channel (10) based on the information obtained by the monitoring device and based on an information about a flow velocity through the channel (10).

9. Device (1) according to claim 8, wherein the device (1) comprises a decision-based ejection device (40) configured
to determine, based on the position of the single particle (17a) within the channel (10) obtained from the calculator (28), information on whether the single particle (17a) is present in a predetermined volume of the channel (10) located near the outlet (12) of the channel (10), and
to eject liquid (16) without the single particle (17a) to a first target position (24a) or with the single particle (17a) to a second target position (24b) dependent on this information.

10. Device (1) according to one of claims 1 to 9, wherein the device (1) comprises
a channel volume monitoring device (26) configured
to monitor a predetermined volume of the channel (10) located near the outlet (12) of the channel (10) and
to obtain information on whether the single particle (17a) is present in the predetermined volume, and
a decision-based ejection device (40) or the decision-based ejection device (40) configured
to eject liquid (16) without the single particle (17a) to a first target position (24a), if the information indicates that the single particle (17a) is not present in the liquid (16) to be output, and
to eject liquid (16) with the single particle (17a) to a second target position (24b), if the information indicates that the single particle (17a) is present in the liquid (16) to be output.

11. Device (1) according to one of claims 1 to 10, wherein the device (1) comprises a particle concentration monitoring device configured
to determine a particle concentration in the liquid (16) including the particles (17) in the reservoir (15) and
to indicate whether the particle concentration exceeds a predetermined threshold.

12. Device (1) according to one of claims 1 to 11, wherein the channel (10) is a continuous filter free channel.

13. Device (1) according to one of claims 1 to 12, wherein the channel (10) is a hose with a uniform diameter over its complete length.

14. Device (1) according to one of claims 1 to 13, wherein the pressure generator (14) is configured to output the single particle (17a) through the outlet (12) of the channel (10) by dispensing the single particle (17a) in a free-flying droplet.

15. Method (100) for particle isolation, comprising the steps
fluidically coupling (110) an inlet of a channel to a reservoir holding a liquid including particles;
causing (120) a relative movement between the particles and the inlet of the channel so as to bring a single particle in a vicinity of the inlet to be aspirated through the inlet;
generating (130) a pressure gradient in the channel by which liquid including the single particle is aspirated from the reservoir through the inlet of the channel against the force of gravity;
outputting (140) liquid through an outlet of the channel.
